# EUROPÄISCHE PATENTSCHRIFT

(11) **EP 3 970 671 B1**
(45) Veröffentlichungstag und Bekanntmachung des Hinweises auf die Patenterteilung: **07.01.2026**
(21) Anmeldenummer: 21196159.4
(22) Anmeldetag: 10.09.2021
(51) Int. Cl.: A61F 5/01, A61F 13/10, A61F 5/30

(54) **HANDMANSCHETTE**
WRIST BRACKET
ATTELLE DE POIGNÉE

(30) Priorität: 21.09.2020 DE 102020124538
(43) Veröffentlichungstag der Anmeldung: 23.03.2022
(73) Patentinhaber: bayer Feinwerk GmbH & Co. KG, 78052 VS-Villingen (DE)
(72) Erfinder: HEITER, Uwe, 78609 Tuningen (DE)
(74) Vertreter: Patentanwälte und Rechtsanwalt Weiß, Arat & Partner mbB

(56) Entgegenhaltungen:
- US-A- 5 865 783
- US-A1- 2003 018 286
- US-A1- 2004 049 141
- US-B1- 6 517 501

## Beschreibung

### Technisches Gebiet

Die Erfindung betrifft eine Handmanschette zur Behandlung des Karpaltunnelsyndroms einer Hand.

### Stand der Technik

Derartige Handmanschetten sind bereits in vielfältiger Form und Ausgestaltung bekannt und gebräuchlich.

So wird beispielsweise in der WO 2003 007 804 A2 sowie der US 2003 / 018 286 A1 eine gattungsgemässe Handmanschette offenbart. Diese Handmanschette kann die linke und die rechte Hand des Benutzers aufnehmen, wobei der Daumen jeweils durch ein seitlich im Gehäuse der Manschette befindliches Loch gesteckt wird.

Ferner ist aus der EP 3 310 306 B1 eine gattungsgemässe Handmanschette bekannt, deren Breite an die Hand des Benutzers angepasst werden kann.

Die gattungsgemässen Handmanschetten, welche nach dem teilweise als Porrata-Prinzip (nach den Erfindern der WO 2003 007 804 A2) bezeichneten Wirkprinzip funktionieren, verfügen seit jeher über ein seitliches Loch zum Herausstrecken des Daumens.

Die Erfinder der vorliegenden Erfindung haben erkannt, dass sämtliche bekannten Handmanschetten zumindest einen der folgenden Nachteile aufweisen: Entweder muss der Daumen aufwändig zunächst in die Manschette eingeschoben und dann durch ein Loch nach aussen gestreckt ("eingefädelt") werden, oder die Breite der Manschette muss veränderlich sein, um ein aufwändiges "Einfädeln" des Daumens zu vermeiden. Die Manschette gemäss der EP 3 310 306 B1 funktioniert nach dem zweitgenannten Prinzip. Die zweitgenannte Variante führt hierbei stets zu einer deutlich komplizierter gebauten Manschette.

Die US 2003 / 018 286 A1 offenbart eine Handmanschette zur Behandlung des Karpaltunnelsyndroms einer Hand mit einem Grundkörper. Der Grundkörper umfasst einen Dorsal-Abschnitt, einen Thenar-Abschnitt sowie einen Hypothenar-Abschnitt, wobei der Dorsal-Abschnitt eingerichtet ist, um an zumindest einer Stelle einen Druck auf die Dorsalseite der Hand auszuüben. Der Thenar-Abschnitt ist gemeinsam mit dem Hypothenar-Abschnitt eingerichtet, auf die Thenar- und die Hypothenar-Region der Palmarseite der Hand einen Druck auszuüben. Der Dorsal-Abschnitt und der Thenar- sowie der Hypothenar-Abschnitt sind gemeinsam eingerichtet, durch Ausübung des Drucks den Karpaltunnel der Hand zu weiten. Die US 2003 / 018 286 A1 offenbart ferner ein Loch, in welches der Daumen sowohl der linken als auch der rechten Hand eingebracht werden kann.

Die US 5 865 783 A offenbart eine medizinische Fingerschiene umfassend eine schlauchförmige Handbandage aus Stoff mit zwei offenen Enden, einem Daumenschlitz und einem herausnehmbaren Einsatzkern.

Die US 6 517 501 B1 offenbart eine Handgelenkstütze mit einem länglichen Körper aus Kunstharz, der einen C-förmigen Querschnitt aufweist. Der Stützkörper kann verstellbar am Handgelenk des Trägers befestigt werden.

Die US 2004 / 049 141 A1 offenbart eine Handgelenkstütze mit einem Körper, der das Handgelenk eines Trägers umschliesst, sowie mit einer palmarseitigen Stütze und einer abnehmbaren dorsalseitigen Stütze.

### Aufgabe der Erfindung

Die Aufgabe der vorliegenden Erfindung ist es, die Nachteile aus dem Stand der Technik zu überwinden.

### Lösung der Aufgabe

Zur Lösung der Aufgabe führt der Gegenstand des Anspruchs 1. Vorteilhafte Ausgestaltungen sind in den Unteransprüchen beschrieben.

Eine erfindungsgemässe Handmanschette zur Behandlung des Karpaltunnelsyndroms einer Hand umfasst einen Grundkörper. Der Grundkörper umfasst einen Dorsal-Abschnitt und einen Palmar-Abschnitt, wobei der Dorsal-Abschnitt eingerichtet ist, um an zumindest einer Stelle einen Druck auf die Dorsalseite der Hand auszuüben. Der Palmar-Abschnitt ist eingerichtet, beidseits des Karpaltunnels jeweils an zumindest einer Stelle einen Druck auf die Palmarseite der Hand auszuüben. Der Dorsal-Abschnitt und der Palmar-Abschnitt sind gemeinsam eingerichtet, um durch die Ausübung des Drucks den Karpaltunnel der Hand zu weiten.

Der Grundkörper weist zwischen dem Dorsal-Abschnitt und dem Palmar-Abschnitt zwei einander im Wesentlichen gegenüberliegende, vorzugsweise einseitig offene Schlitze auf, wobei die Schlitze eingerichtet sind, den Daumen der linken oder der rechten Hand aufzunehmen. Ferner umfasst der Grundkörper vorzugsweise zwischen Dorsal- und Palmar-Abschnitt zwei sich an die Schlitze anschliessenden Seitenteile, welche Dorsal- und Palmar-Abschnitt miteinander verbinden.

Durch diese Schlitze kann auf ein Loch für den Daumen verzichtet werden. Der wesentliche Unterschied zwischen den einseitig offenen Schlitzen gemäss der vorliegenden Erfindung und dem Loch gemäss dem Stand der Technik liegt darin, dass der Daumen bei bekannten Handmanschetten von innen durch das Loch nach aussen gestreckt wird. Der Daumen muss somit zunächst ins Innere der Handmanschette eingebracht werden, um von dort durch das Loch nach aussen gestreckt zu werden. Dies bedingt eine entsprechende breiter geformte Manschette die dann in der Gebrauchslage schlechter sitzt und/oder eine Weitenverstellung erfordert. Gemäss der vorliegenden Erfindung ist es dank der einseitig offenen Schlitze nicht mehr nötig, den Daumen zunächst ins Innere der Handmanschette "einzufädeln".

Die Benennung einzelner Abschnitte der Handmanschette sowie die Beschreibung ihrer Funktion orientieren sich an den anatomischen Lage- und Richtungsbezeichnungen der Hand. Hierbei bedeutet "dorsal-" den Handrücken betreffend oder in Richtung auf den Handrücken zu. Die Bezeichnung "palmar-" bezieht sich entsprechend auf die Hand-Hohlseite bzw. Hand-Innenseite. Weitere derartige Begriffspaare sind "distal" (in Richtung Fingerspitzen oder die Fingerspitzen oder das Fingerspitzen-seitige Ende der Handmanschette betreffend) und "proximal" (in Richtung Handgelenk bzw. das Handgelenk oder das Handgelenk-seitige Ende der Handmanschette betreffend) sowie "thenar" (in Richtung Daumen) und "hypothenar" (in Richtung kleiner Finger).

Hierbei ist zu bemerken, dass die Handmanschette gemäss der vorliegenden Erfindung hinsichtlich "dorsal"/"palmar" sowie "distal"/"proximal" bevorzugt in eindeutiger Weise ausgebildet ist, während dies hinsichtlich "thenar"/"hypothenar" nicht der Fall ist. Um eine nachstehend noch näher beschriebene Gebrauchslage zu erreichen, gibt es vorzugsweise nur hinsichtlich "dorsal"/"palmar" sowie "distal"/"proximal" jeweils genau eine Möglichkeit, wie die Hand des Benutzers in die Handmanschette eintreten kann. Die Hand des Benutzers muss vorzugweise am proximalen Ende eingeschoben werden, wobei die Dorsal- und die Palmar-Seite der Hand korrekt ausgerichtet sein muss.

Dies gilt jedoch nicht für die thenar/hypothenar-Ausrichtung der Hand. Hierdurch wird einer der Vorteile der vorliegenden Erfindung deutlich: Am proximalen Ende können entweder die linke oder die rechte Hand des Benutzers eingeschoben werden. Die Orientierung von Hand und Handmanschette hinsichtlich hypothenar/thenar spielt keine Rolle.

Die Möglichkeit, beide Hände des Benutzers aufnehmen zu können, wird vorzugsweise durch eine im Wesentlichen symmetrische Ausgestaltung der Handmanschette gewährleistet. Die entsprechende Symmetrie-Ebene wird durch die dorsal-palmar verlaufende Achse sowie die distal-proximal verlaufende Achse aufgespannt. Ferner liegt diese Symmetrie-Ebene für Handmanschetten, welche über eine nachstehend noch näher erläuterte eine Öffnung im Palmar-Abschnitt verfügen, im Wesentlichen mittig durch diese Öffnung. Ist ferner ein ebenfalls nachstehend erläuterter Führungssteg vorhanden, so liegt dieser ebenfalls in der Symmetrie-Ebene. Diese spiegelsymmetrische Ausgestaltung der Handmanschette ist anhand der Figuren leicht verständlich. In den Figuren sind die Lage- und Richtungsbezeichnungen teilweise angegeben. Der Anschaulichkeit halber wurde allerdings auf die Einzeichnung der Symmetrie-Ebene verzichtet.

Zusammenfassend lässt sich also sagen, dass die zu behandelnde Hand vorzugsweise am proximalen Ende in die Handmanschette eingeschoben wird, wobei die Dorsal-Seite der Hand mit dem Dorsal-Abschnitt der Handmanschette in Kontakt tritt. Da die Handmanschette keinen vordefinierten Thenar- oder Hypothenar-Abschnitt umfasst, welcher spezifisch zur Aufnahme der Thenar- oder Hypothenar-Region der Hand ausgebildet ist, können beide Hände des Benutzers am proximalen Ende in die Handmanschette eingeschoben werden.

Das grundsätzliche Wirkprinzip, wie der Karpaltunnel durch Druckausübung aufgeweitet wird, ist von den bekannten Handmanschetten zur Behandlung des Karpaltunnelsyndroms her bekannt und wird im Zusammenhang mit den Figuren näher erläutert. Dieses Wirkprinzip sei hier nur kurz geschildert:
Der Palmar-Abschnitt übt auf die Palmar-Seite der Hand einen Druck aus. Die Hand kann diesem Druck nicht ausweichen, da der Dorsal-Abschnitt auch auf die Dorsal-Seite der Hand einen Druck ausübt. Der dorsal auf die Hand ausgeübte Druck wird meist im Wesentlichen flächen- oder linienförmig aufgebracht. Dies bedeutet vorzugsweise, dass der den Druck ausübende bzw. aufbringende Abschnitt entweder flächenförmig oder linienförmig gestaltet ist.

Der Palmar-Abschnitt übt beidseits des Karpaltunnels jeweils einen Druck auf die Palmar-Seite der Hand aus. Diese Drücke werden vorzugsweise linienförmig aufgebracht. Auf der Palmar-Seite sind Angriffs-Punkte (bzw. Angriffs-Linien) und Wirkrichtungen der Drücke vorzugsweise so gewählt, dass der Karpaltunnel geweitet wird. Die Angriffspunkte der Drücke sind vorzugsweise beidseits neben dem Karpaltunnel gelegen, aber nicht weit von diesem beabstandet. Die Wirkrichtungen der Drücke sind vorzugsweise so gewählt, dass die Wirkrichtung des Drucks welcher thenar-seitig neben dem Karpaltunnel auf die Palmar-Seite ausgeübt wird, nach dorsal-thenar orientiert ist. Entsprechend ist der Druck, welcher hypothenar-seitig neben dem Karpaltunnel auf die Palmar-Seite ausgeübt wird, nach dorsal-hypothenar orientiert. Dies wird im Hinblick auf die Figuren näher erläutert.

Die genannten Stellen, an denen die genannten Drücke ausgeübt werden, können punktförmig, streckenförmig oder flächenförmig sein, wobei flächenförmige und linienförmige Stellen bevorzugt sind.

Besonders bevorzugt wird an genau einer Stelle, welche vorzugsweise flächenförmig ist, auf der Dorsalseite Druck ausgeübt. Ebenfalls besonders bevorzugt wird an genau zwei Stellen, welche vorzugsweise linienförmig sind, auf der Palmar-Seite Druck ausgeübt.

Der Dorsal-Abschnitt und der Palmar-Abschnitt können jeweils entweder direkt oder indirekt den beschriebenen Druck ausüben.

Hierbei kann sich die Druck-Ausübung des Palmar-Abschnitts (punkt/strecken/flächenförmig sowie direkt oder indirekt) von derjenigen des Dorsal-Abschnitts unterscheiden.

Eine direkte Druck-Ausübung erfolgt beispielsweise, wenn der Dorsal-Abschnitt und/oder der Palmar-Abschnitt des Grundkörpers unmittelbar mit der zu behandelnden Hand in Kontakt treten.

Eine indirekte Druck-Ausübung erfolgt beispielsweise, wenn zwischen dem/den genannte(n) Abschnitt(en) und der Hand Polster eingelegt werden. Ferner kann auch daran gedacht sein, den Druck verteilende bzw. im Allgemeinen den Druck beeinflussende oder die Richtung des Drucks beeinflussende Einsätze zwischen den Grundkörper und die Hand zu bringen.

Die offenen Schlitze zur Aufnahme der Daumen verlaufen vorzugsweise entlang einer Längsachse der Handmanschette, wobei die Längsachse einer von distal nach proximal verlaufenden Achse entspricht. Die Schlitze sind vorzugsweise einseitig am proximalen Ende der Handmanschette offen, so dass von dort die Hand in die Handmanschette eingeschoben werden kann. In Gebrauchslage ragt der Daumen vom Inneren der Handmanschette durch den Schlitz nach aussen. Der Schlitz weist vorzugsweise ein offenes proximales Ende und ein geschlossenes distales Ende auf, welches als Anschlag für den Daumen und somit als Begrenzung für das Einschieben der Hand dienen kann. Durch die einander gegenüberliegend angeordneten Schlitze können sowohl die rechte als auch die linke Hand in das proximale Ende der Handmanschette eingeschoben werden. Die Schlitze sind vorzugsweise einseitig offene und länglich ausgeführte Ausnehmungen, wobei die offenen Enden der Schlitze vorzugsweise am proximalen Ende der Handmanschette liegen.

Nachdem die Hand vollständig in die Handmanschette eingeschoben wurde, kann man in Bezug auf die Handmanschette und die Hand von einer Gebrauchslage sprechen.

Der Grundkörper bildet vorzugsweise das Gerüst der Handmanschette. Die Handmanschette kann aber auch ausschliesslich aus dem Grundkörper bestehen. Abgesehen von dem Grundkörper kann die Handmanschette noch Polster, Einlagen, aufblasbare und/oder lageveränderliche Elemente wie beispielsweise Druckaufbau-Elemente oder dergleichen umfassen, von denen einige bereits vorstehend erwähnt wurden. Polster und Einlagen können dem Komfort und/oder dem Druckaufbau und/oder einer Ausrichtung des Drucks bzw. dessen Wirkrichtung dienen. Druckaufbau-Elemente sind vorzugsweise Luftkissen oder spezielle Schrauben, welche beim Eindrehen einen Druck auf den gewünschten Abschnitt der Hand aufbauen. Vorzugsweise umfasst die Handmanschette genau ein Druckaufbau-Element.

Die Handmanschette kann zum Aufblasen der Druckaufbau-Elemente beispielsweise mit einem Kompressor oder einem Pumpball verbunden sein. Pumpbälle, insbesondere solche mit Manometer und Ventil, sind robust und für den Einsatz mit der erfindungsgemässen Handmanschette gut geeignet. Pumpbälle mit Manometer und Ventil sind von bekannten manuell bedienbaren Blutdruckmessgeräten her bekannt. Durch Betätigen des Pumpballs wird das Druckaufbau-Element aufgeblasen, wobei der Druck mit dem Manometer kontrolliert wird. Das handbetätigte Ventil dient dazu, die Luft manuell abzulassen. Solche Pumpbälle sind bevorzugt dauerhaft über einen Schlauch mit der Handmanschette verbunden.

Zum Aufblasen der Druckaufbau-Elemente können aber auch andere Einrichtungen zum Pumpen von Luft zum Einsatz kommen. Im Allgemeinen umfasst die Handmanschette vorzugsweise eine solche Einrichtung und ein Manometer zum Anzeigen des Drucks im Druckaufbau-Element. Die vorgenannte Einrichtung und das Manometer können auch direkt auf dem Grundkörper der Handmanschette platziert werden, so dass auf den Schlauch verzichtet werden kann. Dies ist vorteilhaft, wenn Benutzer die Handmanschette bspw. auf Reisen mitnehmen und die zweiteilige, über den Schlauch verbundene Anordnung von Pumpball mit Manometer einerseits und Grundkörper andererseits unpraktisch ist.

Es können auch Einrichtungen zum Pumpen von Luft eingesetzt werden, welche von bekannten vollautomatischen Blutdruckmessgeräten her bekannt sind, bei denen das Aufpumpen nicht händisch erfolgt. In der Regel handelt es sich hierbei um Kompressoren.

Sämtliche Ausführungsbeispiele der vorliegenden Erfindung umfassen die vorstehend beschriebenen Schlitze zur Aufnahme der Daumen von rechter und linker Hand. Dies bewirkt den Vorteil, dass beide Hände mühelos am proximalen Ende in die Handmanschette eingeschoben werden können. Hierfür sind kein Aufklappen, Aufweiten oder dergleichen der Handmanschette und auch kein "Einfädeln" der Hand und insbesondere des Daumens nötig. Die meisten Manschetten aus dem Stand der Technik umfassen ein Loch, durch welches der Daumen der im Inneren der Manschette aufgenommenen Hand nach aussen gestreckt wird. Dies verkompliziert Aufbau und Handhabung bekannter Handmanschetten. Die einseitig offenen Schlitze zur Aufnahme des Daumens gemäss der erfindungsgemässen Manschette überwinden diese Nachteile.

Ferner sei bemerkt, dass auch die vorstehend beschriebene sehr einfache Ausführungsform der vorliegenden Erfindung, welche ohne die nachstehend beschriebenen Luftkissen etc. auskommt und im Wesentlichen oder sogar ausschliesslich aus dem Grundkörper besteht, die gewünschte Karpaltunnel-Weitung bereits zuverlässig bewirken kann. Der hierfür nötige Druck, welcher vorstehend bereits ausführlich beschrieben wurde, kann auf mehrere Weisen erzeugt werden.

Einerseits kann die Handmanschette im Wesentlichen aus einem starren Material gefertigt sein. Der Druck wird in diesem Beispiel schlicht dadurch erzeugt, dass die Hand in die Handmanschette eingeschoben wird, sofern der Abstand zwischen Palmar-Abschnitt und Dorsal-Abschnitt etwas kleiner gewählt ist als der Abstand zwischen Palmar- und Dorsal-Seite der zu behandelnden Hand. Diese wird hierdurch beim Einschieben etwas gequetscht, was den gewünschten Druck bewirkt.

Andererseits kann die Handmanschette zumindest teilweise aus einem flexiblen, d.h. nachgiebigen bzw. elastischen Material gefertigt sein. Auch dann ist der Abstand zwischen Palmar-Abschnitt und Dorsal-Abschnitt etwas kleiner gewählt ist als der Abstand zwischen Palmar- und Dorsal-Seite der zu behandelnden Hand. Beim Einschieben wird der Abstand zwischen Dorsal- und Palmar-Abschnitt der Handmanschette durch die Flexibilität des Materials jedoch vergrössert. Dadurch entsteht eine Rückstellkraft, welche von den Dorsal- und Palmar-Abschnitten der Handmanschette auf die Dorsal- und Palmar-Seite der Hand übertragen wird.

Ein weiterer Vorteil sämtlicher erfindungsgemässer Handmanschetten gegenüber dem Stand der Technik ist, dass ihre Bauweise ohne Verstellung des Grundkörpers oder sonstige Manipulationen grosse, mittelgrosse und kleine Hände aufnehmen kann. Kleine Hände werden schlicht weiter in die Handmanschette eingeschoben als grosse Hände. Bei grossen Händen bestand im Stand der Technik oftmals das Problem, das die Breite der Handmanschette eine Anpassung nötig machte. Diesbezüglich sind abermals die Schlitze der erfindungsgemässen Handmanschette vorteilhaft, da durch einen Schlitz der Daumen nach aussen gestreckt wird und der gegenüberliegende Schlitz bei sehr grossen Händen ein teilweises Hinausstrecken der Hypothenar-Region der Hand erlaubt. Anders ausgedrückt kann bei sehr breiten Händen sowohl der Daumen als auch die dem Daumen gegenüberliegende Region der Hand ein Stück weit aus dem Inneren der Handmanschette durch die Schlitze nach aussen ragen.

Ein **Abstand** zwischen dem Dorsal-Abschnitt und dem Palmar-Abschnitt kann sich von einem distalen Ende zu einem proximalen Ende des Grundkörpers vergrössern.

Der Grundkörper kann sich also zum distalen Ende hin verjüngen. Diese Verjüngung entspricht der Anatomie der Hand, deren dorsal-palmar-Ausdehnung (sozusagen ihre Höhe) nahe am Handgelenk deutlich grösser ist als nahe der Fingerspitzen. Dieser Abstand zwischen Dorsal- und Palmar-Abschnitt kann rechtwinklig zu einer gedachten Mittel-Längsachse der Handmanschette gemessen werden, wobei diese gedachte Mittel-Längsachse die proximal-distal verlaufende Achse ist.

Wenn der Abstand zwischen Dorsal- und Palmar-Abschnitt sich zum distalen Ende hin verkleinert, wird die Hand bereits beim Einschieben vorteilhafterweise durch die vorgenannten Abschnitte mit Druck beaufschlagt. Der Grund ist, dass die Hand zum Handgelenk hin (d.h. an ihrem proximalen Ende) dicker bzw. sozusagen "höher" ist als nahe der Fingerspitzen. Je weiter diese dickeren bzw. höheren Bereiche der Hand in die Manschette geschoben werden, desto grösser wird der Druck, den die Manschette auf sie ausübt.

Bekanntermassen erfolgt die Anwendung des Drucks zwecks Aufweitung des Karpaltunnels mit gattungsgemässen Handmanschetten nahe des Handballens, d.h. am proximalen Ende der Hand. Aus diesem Grund ist die vorstehende Variante mit sich zum distalen Ende der Manschette hin verjüngendem Abstand zwischen Dorsal- und Palmar-Abschnitt vorteilhaft.

Es kann zusätzlich oder alternativ auch daran gedacht sein, eine Breite des Grundkörpers vom distalen Ende zum proximalen Ende zu vergrössern. Diese Verbreiterung kann den gesamten Grundkörper oder nur einen distalen Abschnitt des Grundkörpers betreffen. Die Breite wird in thenar-hypothenar-Richtung gemessen, also orthogonal zur distal-proximalen Achse und zur dorsal-palmaren Achse. Dies entspricht ebenfalls der Anatomie der Hand, deren Breite zu den Fingerspitzen hin abnimmt.

Die Handmanschette kann zumindest einen **Führungssteg** in einem distalen Abschnitt des Grundkörpers umfassen, wobei der zumindest eine Führungssteg eingerichtet sein kann, um in einer Gebrauchslage zwischen zwei Fingern der Hand zu liegen, und somit eine zur Behandlung wirksame Orientierung der Hand zu gewährleisten. Der zumindest eine Führungssteg bewirkt also, dass die Hand und insbesondere deren Karpaltunnel korrekt in der Handmanschette positioniert wird.

Es kann an genau einen Führungssteg gedacht sein, welcher in Gebrauchslage zwischen dem Mittelfinger und dem Ringfinger der zu behandelnden Hand liegt. Selbstverständlich kann auch daran gedacht sein, dass der Führungssteg zwischen Mittelfinger und Zeigefinger oder zwischen dem kleinen Finger und dem Ringfinger liegt. Es kann auch daran gedacht sein, zwei oder alle drei der wie vorstehend beschrieben angeordneten Führungsstege vorzusehen.

Die Ausdehnung des zumindest einen Führungsstegs in Längsrichtung, d.h. entlang der distal-proximal verlaufenden Achse, ist vorzugsweise so bemessen, dass er kürzer ist als der Zwischenraum zwischen den betreffenden Fingern. Vorzugsweise dient bereits der oben beschriebene Schlitz des Grundkörpers als Anschlag für den Daumen und begrenzt bereits ausreichend das Einschieben der Hand in die Handmanschette. Es kann jedoch auch daran gedacht sein, dass der zumindest eine Führungssteg als zusätzlicher Anschlag und somit als Begrenzung für das Einschieben der Hand in die Handmanschette genutzt wird.

Es kann auch daran gedacht sein, dass der zumindest eine Führungssteg entlang der distal-proximal verlaufenden Achse längenveränderlich ist. Der zumindest eine Führungssteg kann beispielsweise einfahrbar oder komprimierbar ausgestaltet sein. Dadurch kann ein sicheres Positionieren unterschiedlich grosser Hände zusätzlich verbessert werden. Eine derartige Ausgestaltung kann in einigen Ausführungsvarianten / Ausführungsformen vorteilhaft sein, ist aber nicht zwingend nötig.

Der Grundkörper kann im Profil im Wesentlichen einem abgeflachten Kreis, einem abgeflachten Oval oder einem abgeflachten C gleichen.

Der Grundkörper kann derart **federnd** eingerichtet sein, dass sich beim Einschieben der Hand der Abstand zwischen dem Dorsal-Abschnitt und dem Palmar-Abschnitt vergrössert, wodurch in Gebrauchslage der Druck zur Weitung des Karpaltunnels bereitgestellt wird. Der Dorsal-Abschnitt und/oder der Palmar-Abschnitt wirken somit als Federn, die beim Einschieben durch die Hand selbst vorgespannt werden. Die Rückstellkraft dieses zumindest einen federnden Abschnitts stellt dann den Druck zur Weitung des Karpaltunnels bereit. In diesem Fall kann auf separate Druckaufbau-Elemente verzichtet werden.

Alternativ kann der Grundkörper, wie weiter oben bereits erläutert, starr ausgebildet sein, so dass weder der Dorsal-Abschnitt noch der Palmar-Abschnitt federnd wirken. In diesem Fall kann der Druck bereitgestellt werden, indem die Hand unter entsprechendem Druck in die Handmanschette eingeschoben wird. Hierbei wird nach Erreichen der Gebrauchslage beispielsweise auf Grund einer Formgebung vom Palmar- und Dorsal-Abschnitt sowie der beim Einschieben bewirkten Kompression der Hand der nötige Druck bereitgestellt. Auch in diesem Fall kann auf ein separates Druckaufbau-Element verzichtet werden.

Natürlich können die vorgenannten Ausführungsformen auch mit Druckaufbau-Elementen ausgestattet sein. Diese können alternativ oder zusätzlich zu den vorstehend beschriebenen Möglichkeiten des Druckaufbaus zum Einsatz kommen.

Durch die vorstehend beschriebene federnde Ausgestaltung des Grundkörpers erfolgt also ein besonders einfacher und effizienter Druckaufbau, wobei keine weiteren Hilfsmittel wie beispielsweise Luftkissen nötig sind. Selbstverständlich ist es aber möglich, den federnden Aufbau in Kombination mit einem nachstehend noch näher beschriebenen Luftkissen oder dergleichen einzusetzen.

Der Palmar-Abschnitt kann einen ersten und einen zweiten **Hypo-/Thenar-Abschnitt** umfassen, wobei sich der Abstand zwischen den beiden Hypo-/Thenar-Abschnitten verändern kann. Je nachdem, ob die linke oder die rechte Hand in die Handmanschette gesteckt wird, fungiert jeweils einer der beiden Hypo-/Thenar-Abschnitte als Thenar-Abschnitt und der gegenüberliegende Abschnitt als Hypothenar-Abschnitt. Derjenige Hypo-/Thenar-Abschnitt, welcher den Daumen des Benutzers aufnimmt, fungiert als Thenar-Abschnitt. Der gegenüberliegende Hypo-/Thenar-Abschnitt fungiert entsprechend als Hypothenar-Abschnitt.

Der als Thenar-Abschnitt fungierende Hypo-/Thenar-Abschnitt liegt sozusagen daumenseitig seitlich des Karpaltunnels. Der als Hypothenar-Abschnitt fungierende Hypo-/Thenar-Abschnitt liegt auf der gegenüberliegenden Seite seitlich des Karpaltunnels.

Hierbei ist zu bemerken, dass beide Hypo-/Thenar-Abschnitte vorzugsweise beidseits unmittelbar neben dem Karpaltunnel einen (vorzugsweise linienförmigen) Druck auf die Hand ausüben. Die Bezeichnungen "thenar" und "hypothenar" deuten nur an, auf welcher Seite des Karpaltunnels der betreffende Abschnitt liegt, welcher den Druck ausübt. Beide befinden sich dennoch im Wesentlichen mittig und stehen in Gebrauchslage mit dem Palmar-Abschnitt der Hand in Kontakt.

Die Wirkrichtungen des Drucks, den der Hypothenar-Abschnitt auf den entsprechenden Bereich der Palmar-Seite ausübt, verläuft sowohl nach dorsal als auch nach hypothenar, also sozusagen schräg zur Aussenkante der Hand hin, an welcher der kleine Finger liegt. Entsprechend verläuft die Wirkrichtung des Drucks, welchen der Thenar-Abschnitt auf den entsprechenden Bereich der Palmar-Seite ausübt sozusagen schräg zur gegenüberliegenden Aussenkante der Hand hin, d.h. nach dorsal-thenar.

Dass sich der Abstand zwischen den beiden Hypo-/Thenar-Abschnitten beim Einschieben der Hand vergrössern kann, bewirkt eine sehr effiziente Druckausübung zur Aufweitung des Karpaltunnels. Insbesondere stellt die Möglichkeit der Abstandsvergrösserung sicher, dass der Druck mit optimaler Wirkrichtung auf die Palmar-Region der Hand beidseits des Karpaltunnels ausgeübt wird.

Die Möglichkeit einer Vergrösserung des Abstands zwischen den Hypo-/Thenar-Abschnitten kann auf verschiedene Arten bereitgestellt werden. Einerseits kann eine längs, d.h. entlang der distal-proximalen Achse verlaufende Öffnung zwischen dem ersten und dem zweiten Hypo-/Thenar-Abschnitt gedacht sein. Diese Öffnung kann schlitzförmig sein. Sie ist weiter unten näher beschrieben. Andererseits kann auch an eine flexible Region gedacht sein, welche den ersten und den zweiten Hypo-/Thenar-Abschnitt verbindet und die Vergrösserung des Abstands beider Abschnitte zulässt. Alternativ könnte beispielsweise auch an eine Nut gedacht sein, deren Öffnung ins Innere der Handmanschette weist und die die vorbeschriebene Veränderung des Abstands erlauben kann.

Vorzugsweise führt der Aufbau eines Drucks auf die Hand, welcher entweder durch das Einschieben der Hand oder durch entsprechende Druckaufbau-Elemente wie Luftkissen bewirkt wird, zu einer Vergrösserung des Abstands zwischen dem ersten und dem zweiten Hypo-/Thenar-Abschnitt. Hierfür ist in der Regel eine gewisse Flexibilität der vorgenannten Abschnitte nötig. Die Vergrösserung des Abstands zwischen den Hypo-/Thenar-Abschnitten, während diese bereits beidseits des Karpaltunnels eng an der Palmarseite der Hand anliegen, in Verbindung mit der Rückstellkraft, welche durch die Vergrösserung des Abstands zwischen Dorsal- und Palmar-Abschnitt hervorgerufen wird, bewirken letztlich den Druck, welcher den Karpaltunnel wie gewünscht weitet.

In Ausführungsbeispielen ohne den vorbeschriebenen veränderlichen Abstand zwischen den Hypo-/Thenar-Abschnitten umfasst der Palmar-Abschnitt vorzugsweise Aufbauten oder speziell geformte Abschnitte, welche hinsichtlich Wirkrichtung und Angriffspunkt der Kräfte bzw. Drücke dafür sorgen, dass die Aufweitung des Karpaltunnels auch ohne Vergrösserung des Abstands zwischen den Hypo-/Thenar-Abschnitten stattfindet und die gewünschte Wirkrichtung des Drucks auf die Palmarseite der Hand erreicht wird.

Die Vergrösserung des Abstands zwischen den Hypo-/Thenar-Abschnitten kann bereits die gewünschte Wirkung hervorrufen, wenn sie im Bereich von wenigen Millimetern oder sogar im Bereich weniger Zehntel Millimeter liegt. Der Bereich von wenigen Millimetern ist hierbei bevorzugt.

Die Handmanschette kann eine **Öffnung** zwischen den Hypo-/Thenar-Abschnitten aufweisen. Diese Öffnung kann beispielsweise in Form des oben beschriebenen Schlitzes ausgestaltet sein. Alternative Ausgestaltungen der Öffnung sind denkbar.

Die Öffnung kann durchgehend sein, so dass die Handmanschette im Profil im Wesentlichen eine C-Form aufweist. Die Öffnung verläuft entlang der Achse distal-proximal über die gesamte Länge der Handmanschette hinweg.

Ist die Öffnung nicht durchgehend, so kann nur ein Abschnitt, welcher die nicht durchgehende Öffnung beherbergt, im Profil im Wesentlichen eine C-Form aufweisen. Dies ist beispielsweise der Fall, wenn die Öffnung nur in einem proximalen Abschnitt der Handmanschette vorhanden ist, und dort die oben beschriebene Funktion der Hypo-/Thenar-Abschnitte gewährleistet. Wenn zugleich im distalen Abschnitt keine Öffnung vorhanden ist sorgt dies zugleich für eine hohe Stabilität der Handmanschette. Durch die Länge der schlitzförmigen Öffnung kann beispielsweise bestimmt werden, wie weit sich die Hypo-/Thenar-Abschnitte auseinanderbewegen können und/oder welche Kraft dafür überwunden werden muss. Indirekt kann somit durch die Länge der vorbeschriebenen Öffnung eine Krafteinleitung in die Hand gesteuert werden.

Alternativ kann durch die Auswahl eines Materials, aus welchem die Handmanschette gefertigt ist, bestimmt werden, wie weit sich die Hypo-/Thenar-Abschnitte auseinanderbewegen können und/oder welche Kraft dafür überwunden werden muss. Insbesondere eine Elastizität des Materials ist hierbei von Bedeutung, da durch Verbiegen elastischer Hypo-/Thenar-Abschnitte eine erwünschte Rückstellkraft erzeugt werden kann. Ferner können auch der Aufbau der Handmanschette, beispielsweise eine Dicke des Grundkörpers und/oder auf dem Grundkörper angebrachte Verstärkungsrippen diese Funktion übernehmen.

Ein zusätzlicher Vorteil der Öffnung besteht darin, dass sie eine einfache Sichtkontrolle durch den Benutzer erlaubt. Da die beiden Hypo-/Thenar-Abschnitte beidseits des Karpaltunnels positioniert werden, liegt die Öffnung in Gebrauchslage unmittelbar oberhalb des Karpaltunnels (wenn die Palmar-Region der Hand betrachtet wird). Der Benutzer kann daher die Handmanschette korrekt positionieren, indem er einfach sicherstellt, dass die Öffnung oberhalb des Karpaltunnels liegt. Anhand des charakteristischen Verlaufs der von aussen sichtbaren Sehnen, Muskeln und Linien auf der Palmar-Seite der Hand kann der Benutzer auf einfache Weise selbst feststellen, wo bei seiner Hand der Karpaltunnel liegt und somit auch das vorbeschriebene korrekte Positionieren einfach selbst durchführen.

Die Handmanschette kann ein **Druckaufbau-Element** am Dorsal-Abschnitt umfassen, wobei das Druckaufbau-Element eingerichtet ist, an zumindest einer Stelle Druck auf die Dorsalseite der Hand auszuüben. Bei dem Druckaufbau-Element kann es sich beispielsweise um eine Schraube, um ein Luftkissen oder dergleichen handeln. Derartige Druckaufbau-Elemente sind aus dem Stand der Technik bekannt und werden in bekannten Handmanschetten bereits eingesetzt.

Der Grundkörper der Handmanschette kann einstückig gestaltet sein. Dies vereinfacht sowohl die Herstellung als auch den Gebrauch.

Der Grundkörper ist vorzugsweise aus einem Kunststoff oder einem anderen elastischen Material gefertigt.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
Figur 1 eine schematische Ansicht einer ersten Ausführungsvariante einer Handmanschette 1 gemäss der vorliegenden Erfindung sowie
in den Figuren 2 bis 12 verschiedene Ansichten einer zweiten Ausführungsvariante einer Handmanschette 1 gemäss der vorliegenden Erfindung.
Figur 13 zeigt eine dritte, einfache Ausführungsform einer Handmanschette 1.
Figur 14 zeigt eine vierte Ausführungsform einer Handmanschette 1.
Figur 15 zeigt die zweite Ausführungsform einer Handmanschette 1 gemäss Figur 5 mit einem Pumpball 29.

Die Figuren 16 und 17 zeigen eine fünfte Ausführungsform einer Handmanschette 1 mit einer in den Grundkörper 3 integrierten Pumpe 32.

Der besseren Übersicht halber sind nicht in sämtlichen Figuren alle Bezugsziffern eingezeichnet.

### Ausführungsbeispiel

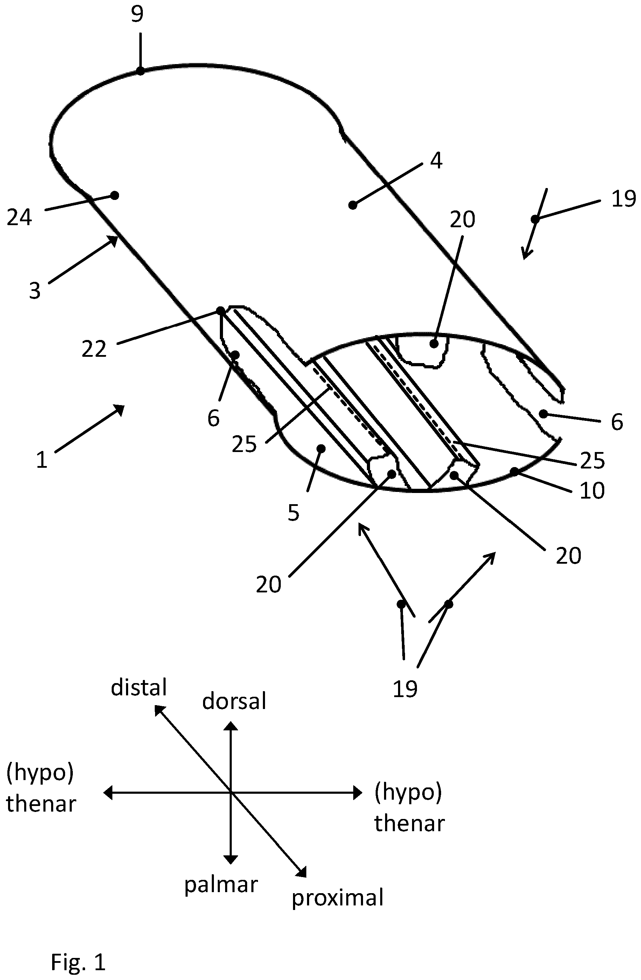

In Figur 1 ist eine Handmanschette 1 dargestellt. In Figur 1 sind ein Grundkörper 3 mit einem Dorsal-Abschnitt 4 und einem Palmar-Abschnitt 5 sowie dazwischen liegenden Schlitzen 6 und Seitenteilen 24 zu erkennen. Ferner sind ein distales Ende 9 und ein proximales Ende 10 angedeutet. Im Inneren des Grundkörpers 3 sind speziell geformte Abschnitte 20 zu erkennen, deren Funktion nachstehend noch genauer erläutert wird.

Ferner ist ein distales Schlitz-Ende 22 erkennbar, welches als Anschlag für einen Daumen 7 (nicht dargestellt) dienen kann oder zumindest eine EinschubTiefe der zu behandelnden Hand 2 begrenzt.

Ferner ist neben Figur 1 ein Koordinatensystem eingezeichnet, um die Richtungen und Achsen (distal, proximal, dorsal, palmar sowie (hypo)-thenar in Bezug auf die in Figur 1 gezeigte Handmanschette 1 zu illustrieren.

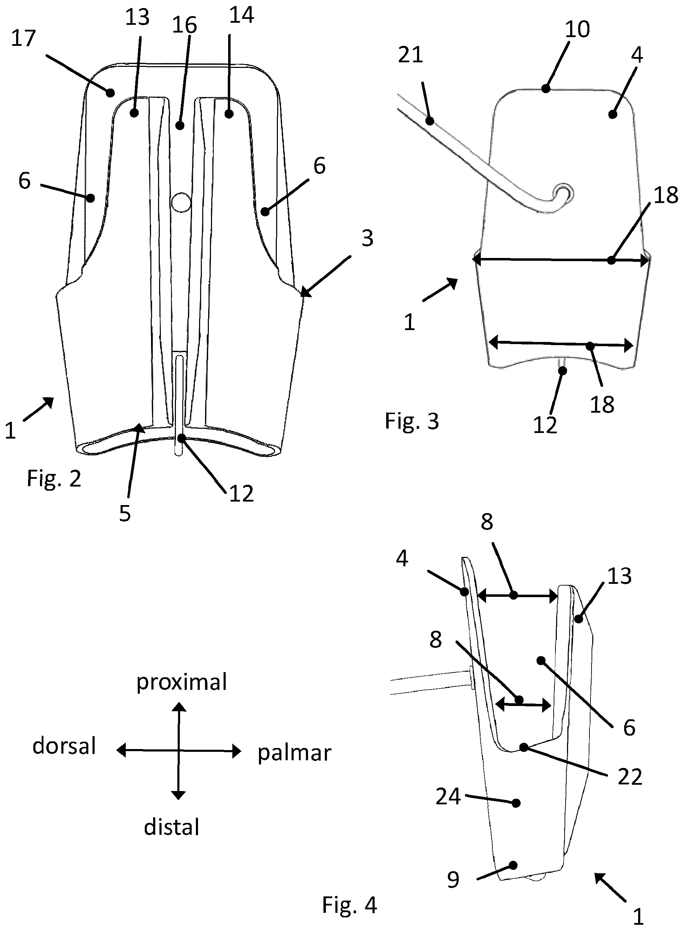

Figur 2 zeigt eine Ansicht von unten auf eine zweite Ausführungsvariante einer Handmanschette 1. Ein Palmar-Abschnitt 5 umfassend einen ersten Hypo-/Thenar-Abschnitt 13 und einen zweiten Hypo-/Thenar-Abschnitt 14 sind ebenfalls zu erkennen. Ferner ist ein Druckaufbau-Element 17 in Form eines Luftkissens sowie ein Führungssteg 12 zu erkennen. Seitlich sind die Schlitze 6 erkennbar. Ferner ist eine distal-proximal verlaufende Öffnung 16 zwischen den beiden Hypo-/Thenar-Abschnitten 13, 14 erkennbar.

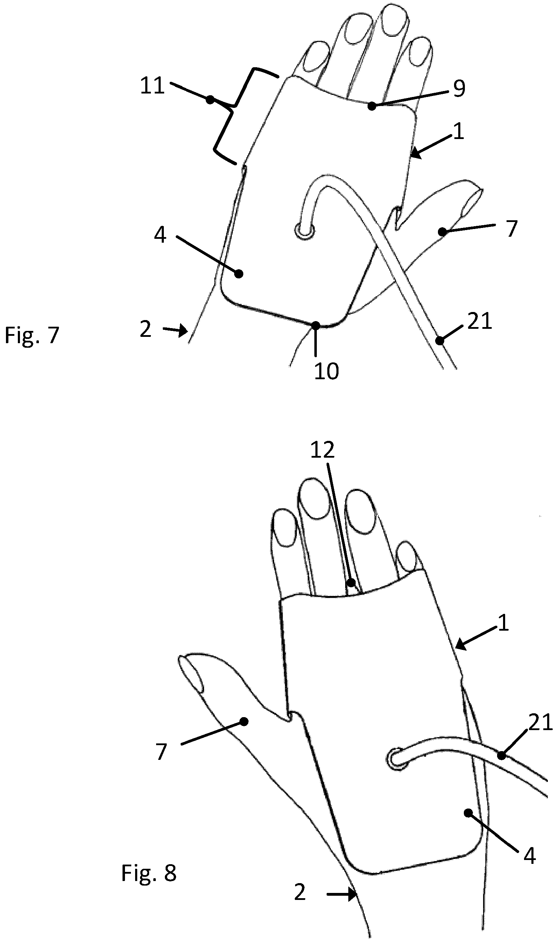

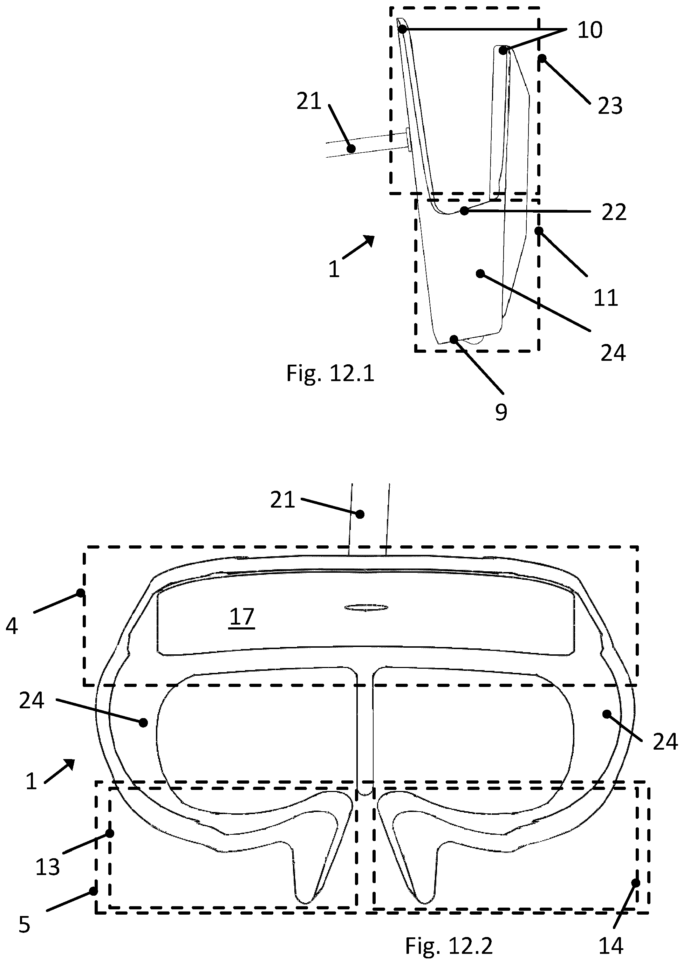

Figur 3 zeigt eine Ansicht auf den Dorsal-Abschnitt 4 der Handmanschette 1 aus Figur 2. Ferner ist an zwei Stellen eine Breite 18 der Handmanschette 1 eingezeichnet. Ferner ist ein Teil eines das Luftkissen 17 versorgenden Schlauchs 21 erkennbar. Aus Figur 3 geht hervor, dass nur die Breite 18 des distalen Abschnitts 11 (vgl. Figuren 7 und 12.1), welcher die Hand 2 des Benutzers vollständig auch mittels noch zu beschreibender Seitenteile 24 umschliesst, zum proximalen Ende 10 hin grösser wird. Der proximale Abschnitt 23 des Grundkörpers 3, welcher seitlich die Schlitze 6 umfasst, verbreitert sich nicht zum proximalen Ende 10 hin.

Figur 4 zeigt eine seitliche Ansicht auf die Handmanschette 1 aus Figur 2. Ferner sind in Bezug auf Figur 4 die Richtungen bzw. Achsen (proximal und distal sowie dorsal und palmar) angegeben. Ferner ist an zwei Stellen ein Abstand 8 zwischen Dorsal- und Palmar-Abschnitt 4, 5 eingezeichnet.

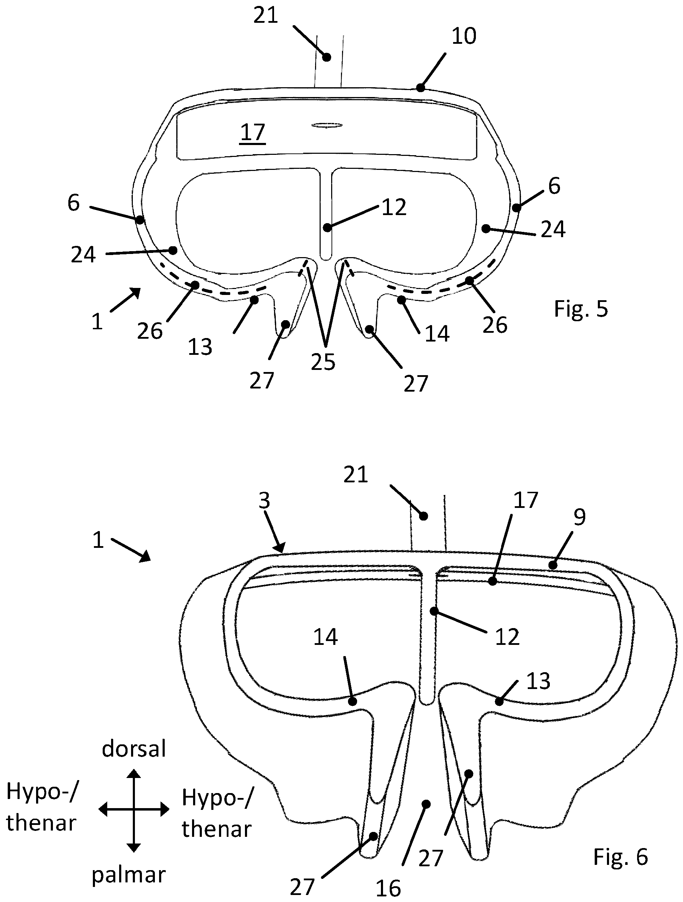

Figur 5 zeigt eine Ansicht auf das proximale Ende 10 der Handmanschette 1 aus Figur 2.

Figur 6 zeigt eine Ansicht auf das distale Ende 9 der Handmanschette 1 aus Figur 2. In Bezug auf Figur 6 sind die Richtung bzw. Achsen dorsal und palmar sowie die beiden hypo-/thenar-Richtungen bzw. Achsen angegeben.

In Figur 7 ist eine Ansicht auf den Dorsal-Abschnitt 4 der Handmanschette 1 aus Figur 2 gezeigt, in der sich die linke Hand 2 eines Benutzers befindet. Der Daumen 7 der Hand 2 ist erkennbar. Ferner ist auch ein distaler Abschnitt 11 der Handmanschette 1 gekennzeichnet.

In Figur 8 ist eine Ansicht auf den Dorsal-Abschnitt 4 der Handmanschette 1 aus Figur 2 gezeigt, in der sich die rechte Hand 2 eines Benutzers befindet.

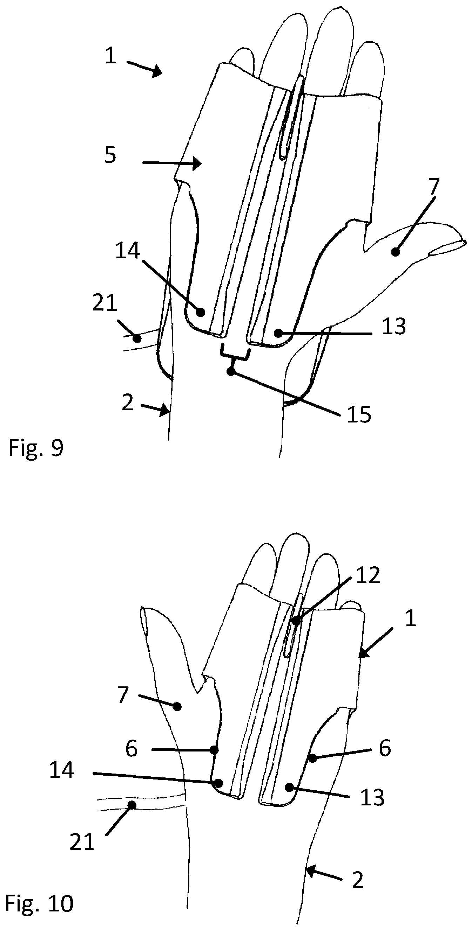

In Figur 9 ist eine Ansicht auf den Palmar-Abschnitt 5 der Handmanschette 1 aus Figur 2 gezeigt, in der sich die rechte Hand 2 eines Benutzers befindet. Ferner ist in Figur 9 ein Abstand 15 zwischen den beiden Hypo/Thenar-Abschnitten 13, 14 eingezeichnet.

In Figur 10 ist eine Ansicht auf den Palmar-Abschnitt 5 der Handmanschette 1 aus Figur 2 gezeigt, in der sich die linke Hand 2 eines Benutzers befindet.

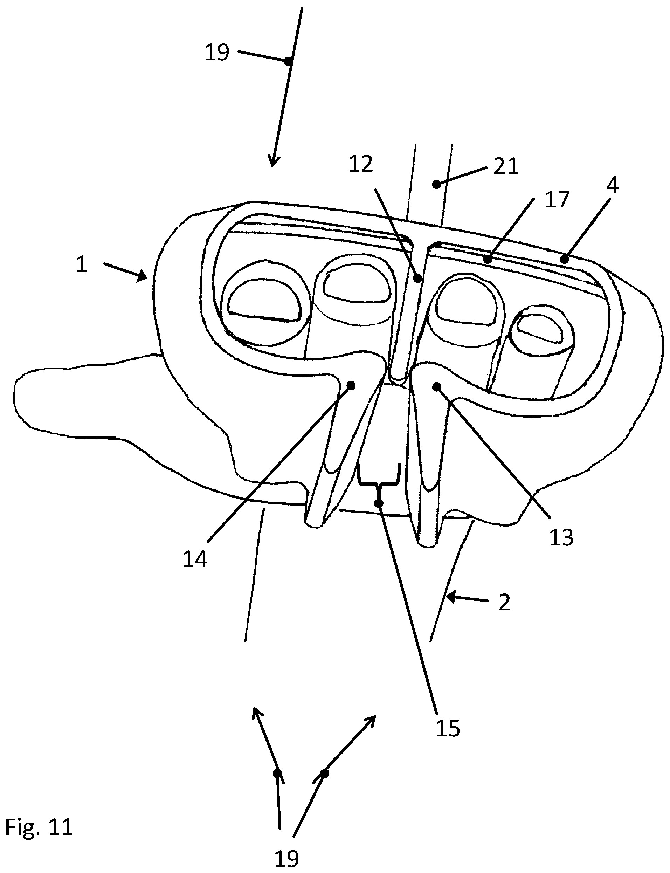

In Figur 11 ist Ansicht auf das distale Ende 9 der Handmanschette 1 aus Figur 2 gezeigt, in der sich die linke Hand 2 eines Benutzers befindet. In Figur 11 sind Hand 2 und Handmanschette 1 zu einem Zeitpunkt während des Einschiebens der Hand 2 dargestellt, bevor die Hand 2 vollständig in die Handmanschette 1 eingetreten ist. die Dorsalseite der Hand 2 hat noch keinen Kontakt zum Druckaufbau-Element 17.

In den Figuren 12.1 und 12.2 sind Ansichten der Handmanschette gemäss den Figuren 4 und 5 gezeigt, um Lage und Ausdehnung der verschiedenen Abschnitte 4, 5, 11, 24, 13, 14 zu illustrieren.

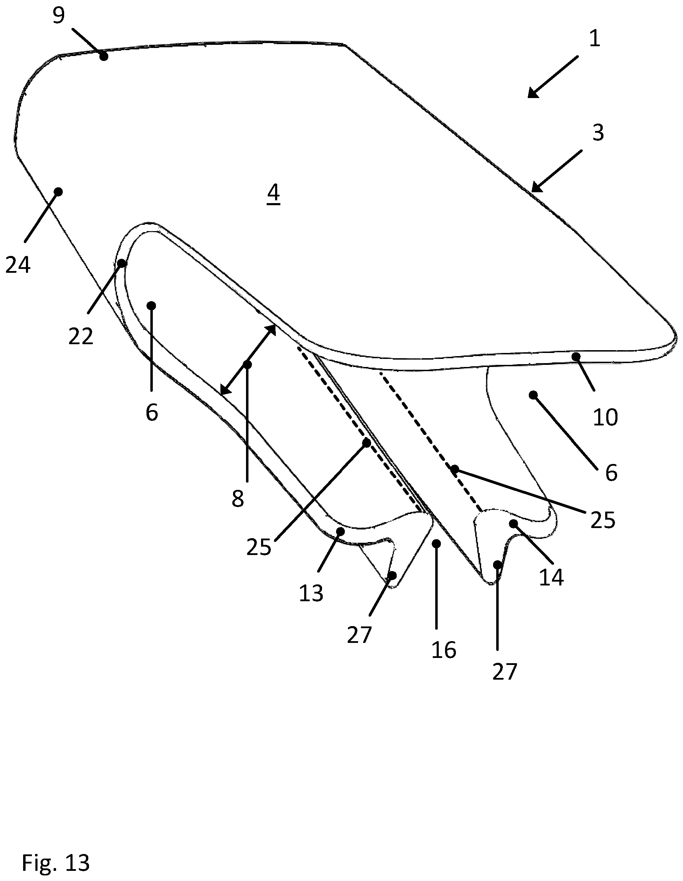

Die Handmanschette 1 gemäss Figur 13 kommt ohne Luftkissen aus und besteht ausschliesslich aus dem Grundkörper 3.

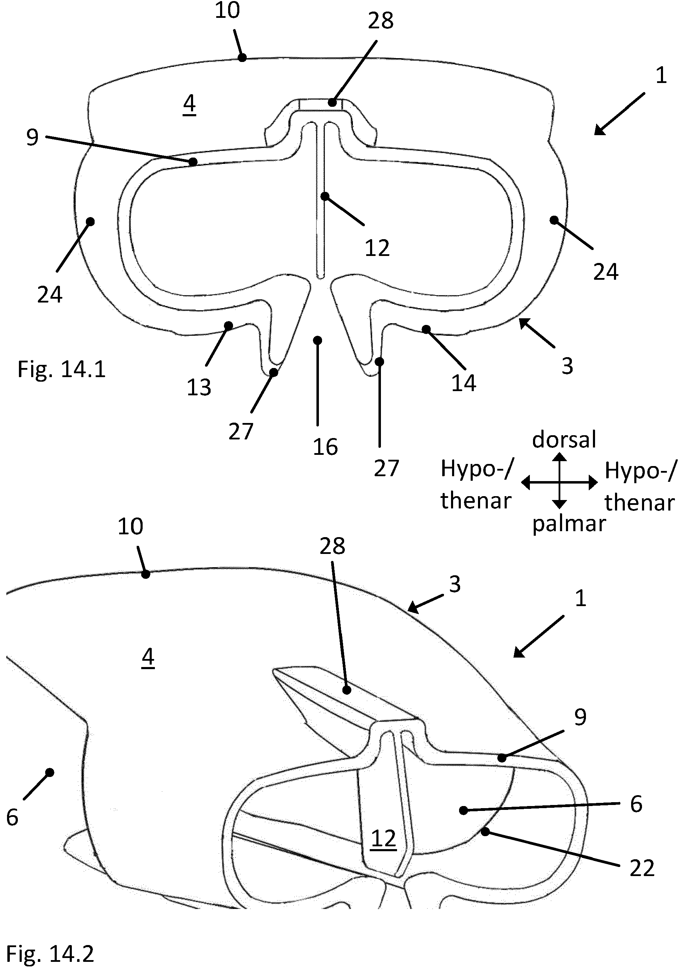

Die Handmanschette 1 gemäss Figur 14 umfasst einen flexiblen Führungssteg 12. Um diesen besser darzustellen, zeigt Figur 14.2 einen Ausschnitt einer Handmanschette 1 nach Figur 14.1 aus einer leicht veränderten Perspektive.

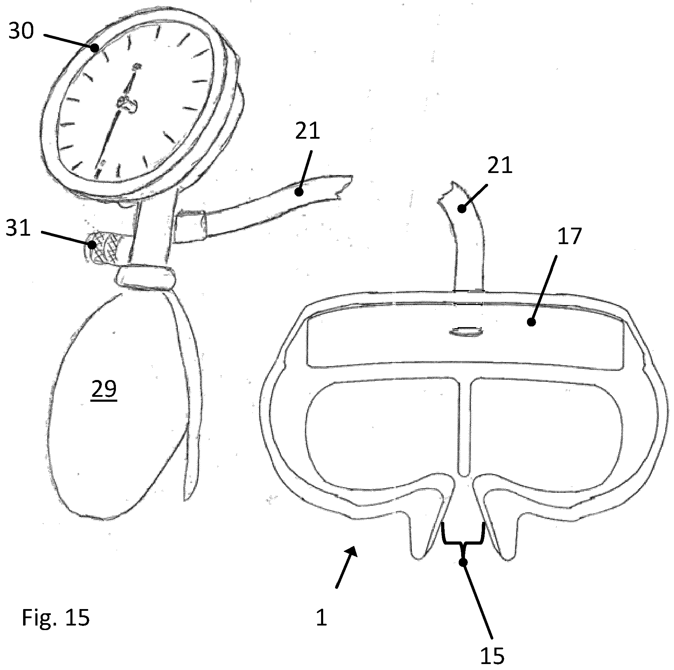

Figur 15 zeigt die Handmanschette 1 nach Figur 5, wobei der Übersicht halber auf die meisten Bezugsziffern verzichtet wurde. Figur 15 illustriert, wie diese Handmanschette 1 über den Schlauch 21 mit einem Pumpball 29 verbunden ist, welcher wiederum mit einem Manometer 30 und einem durch Drehen handbetätigten Ventil 31 ausgestattet bzw. verbunden ist. Der Schlauch 21, welcher Pumpball 29 und Handmanschette 1 verbindet, ist der Übersicht halber nicht vollständig dargestellt.

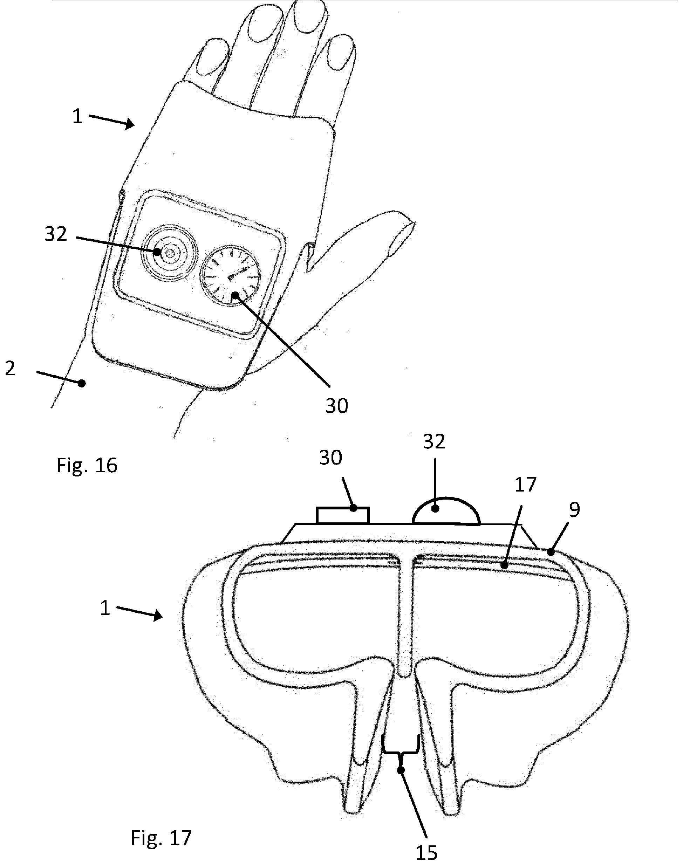

In den Figuren 16 und 17 ist eine alternative Ausführungsform einer Handmanschette 1 gezeigt, welche eine integrierte Pumpe 32 und ein ebenfalls integriertes Manometer 30 aufweist. Die Ansicht der Handmanschette 1 in Figur 17 entspricht derjenigen in Figur 6. Der Übersicht halber wurde auf die meisten Bezugsziffern verzichtet.

Figur 16 zeigt die Handmanschette 1 nach Figur 17 im betriebsbereiten Zustand, nachdem ein Benutzer die Hand 2 hineingesteckt hat. Bezugnehmend auf die Figuren 1 bis 17 erklärt sich die Funktionsweise der erfindungsgemässen Vorrichtung folgendermassen:
Das proximale Ende 10 der Handmanschette 1 kann entweder die linke oder die rechte Hand 2 eines Benutzers aufnehmen, ohne dass der Daumen 7 hierfür aufwändig in ein Loch eingefädelt werden muss. Stattdessen wird beim Einbringen der Hand 2 in die Handmanschette 1 schlicht der Daumen 7 durch einen der beiden Schlitze 6 nach aussen gestreckt.

Die in Figur 1 gezeigte sehr einfach gestaltete Ausführungsvariante ist im Profil oval geformt und umfasst keine Öffnung 16. Ferner sind die Breite und der Abstand zwischen dem Dorsal- und dem Palmar-Abschnitt 4, 5 entlang einer gedachten distal-proximal verlaufenden Achse identisch bzw. unveränderlich. Beim Einschieben der Hand 2 (nicht gezeigt), wird der Karpaltunnel zwischen den beiden speziell geformten Abschnitten 20 platziert, welche sich auf dem Palmar-Abschnitt 5 befinden. Da der proximale Abschnitt der Hand 2 in dorsal-palmar-Richtung höher ist als der distale Abschnitt der Hand, wird die Hand 2 während des Einschiebens in die Handmanschette 1 zunehmend von den speziell geformten Abschnitten 20 sowohl von dorsaler als auch von palmarer Seite mit Druck beaufschlagt. Dieser Druck bewirkt auf die von gattungsgemässen Handmanschetten bekannte Weise eine Aufweitung des Karpaltunnels. Die Wirkrichtung des Drucks, welcher von den beiden speziell geformten Abschnitten 20 auf dem Palmar-Abschnitt 5 der Handmanschette ausgeübt wird, ist durch Pfeile 19 gekennzeichnet.

Die Handmanschette 1 gemäss den Figuren 2 bis 12 und 15 unterscheidet sich in einigen Details von der sehr einfach gebauten Handmanschette 1 nach Figur 1.

In der Handmanschette 1, welche in den Figuren 2 bis 12 und 15 gezeigt ist, wird die Hand 2 ebenfalls in das proximale Ende 10 der Handmanschette 1 eingeschoben.

Wie in Figur 4 erkennbar ist, verringert sich der Abstand 8 zwischen dem Dorsal-Abschnitt 4 und dem Palmar-.Abschnitt 5 zum distalen Ende 9 hin. Dies trägt bereits für sich genommen dazu bei, dass mit zunehmendem Einfahren der Hand 2 in die Handmanschette 1 der gewünschte Druck auf die Palmar-Region der Hand 2 aufgebaut wird.

Weiterhin vergrössert sich beim Einfahren der Hand 2 durch den von der Hand 2 auf den Grundkörper 3 ausgeübten Druck der Abstand 15 zwischen den Hypo-/Thenar-Abschnitten 13, 14, was ebenfalls bereits für sich genommen zu dem gewünschten Druck auf die Palmar-Region der Hand 2 zwecks Weitung des Karpaltunnels beiträgt.

Ausserdem umfasst die Handmanschette 1 gemäss den Figuren 2 bis 12 und 15 ein Druckaufbau-Element 17 in Form eines Luftkissens, welches über einen Kompressor oder von Hand aufgeblasen werden kann. Für ein Aufblasen von Hand kann insbesondere ein von Blutdruckmessgeräten her bekannter Ball ("Pumpball") zum Einsatz kommen, wie dies in Figur 15 gezeigt ist. Auch eine alternative Ausführungsform gemäss den Figuren 16 und 17 ist denkbar. Die Luft wird in beiden Fällen über den Schlauch 21 in das Luftkissen 17 eingeleitet. Hierdurch wird abermals ein Druck auf die Kontakt-Abschnitte zwischen Hand 2 und Handmanschette 1 aufgebaut, wobei diese Kontakt-Abschnitte das am Dorsal-Abschnitt 4 befestigte Luftkissen 17 sowie die den Palmar-Abschnitt 5 bildenden Hypo-/Thenar-Abschnitte 13, 14, und dort vorzugsweise die nachstehend noch erläuterten Kontaktlinien 25, sind. Beim Aufblasen des Luftkissens 17 verringert sich das innere Volumen der Handmanschette 1, was letztlich dazu führt, dass die Hand 2 aus dorsaler und palmarer Richtung mit Druck beaufschlagt wird. Der auf der Dorsal-Seite bzw. in der Dorsal-Region der Hand 2 wirkende Druck verläuft hierbei im Wesentlichen von dorsal nach palmar.

Der auf der Palmar-Seite bzw. in der Palmar -Region der Hand 2 wirkende Druck verläuft hierbei schräg. Er umfasst somit jeweils eine Komponente, welche in Richtung palmar nach dorsal wirkt sowie eine Komponente, welche in Richtung nach (hypo)thenar wirkt. Dieser Sachverhalt ist durch die Wirkrichtungen 19 in den Figuren 1 und 11, jeweils oberhalb bzw. unterhalb des Dorsal- bzw. Palmar-Abschnitts 4, 5 angedeutet. Hierbei wird die gewünschte Wirkrichtung bei der in Figur 1 gezeigten Handmanschette 1 durch die Orientierung der speziell geformten Abschnitte 20 sichergestellt. Bei der in den Figuren 2 bis 12 und 15 bis 17 gezeigten Handmanschette 1 wird die Wirkrichtung hingegen zusätzlich und vorzugsweise hauptsächlich dadurch bereitgestellt, dass sich der Abstand 15 während des Druckaufbaus vergrössert. Da die beiden Hypo-/Thenar-Abschnitte 13, 14 bzw. insbesondere die Kontaktlinien 25 während des Druckaufbaus vorzugsweise bereits in Kontakt mit der Palmar-Region der Hand 2 getreten sind, wird der Karpaltunnel effektiv geweitet, wenn sich der Abstand 15 vergrössert. Derselbe Effekt kann bei den Ausführungsformen gemäss den Figuren 13 und 14 während des Einschiebens der Hand 2 in den Grundkörper 3 erzielt werden.

Die Ansicht gemäss Figur 5 zeigt deutlich, dass das Luftkissen 17 durch seine flächige Gestalt vorzugsweise einen flächigen Druck auf die Dorsal-Seite der Hand 2 aufbringt. Demgegenüber bringen die beiden gedachten Kontaktlinien 25 der Hypo-/Thenar-Abschnitte 13, 14 durch ihre lineare Erstreckung sozusagen einen linear (im Gegensatz zu flächig) wirkenden Druck auf die Palmar-Seite der Hand auf.

In Figur 5 ist gut erkennbar, dass die Hypo-/Thenar-Abschnitte 13, 14 jeweils einen im Querschnitt konvexen Abschnitt 26 umfassen. Auf diese Weise wird sichergestellt, dass beim Einschieben der Hand (ab Figur 7 gezeigt) der Kontakt zwischen Hand und Palmar-Abschnitt 5 ausschliesslich oder zumindest vorrangig über die (gedachten) Kontaktlinien 25 erfolgt, und diese an den für die Behandlung des Karpaltunnelsyndroms wirksamen Stellen den gewünschten Druck einleiten. Der Palmar-Abschnitt 5 ist also so gestaltet, dass nur linienförmige Abschnitte, angedeutet durch die gedachten Kontaktlinien 25, wirksam Druck in die Hand einleiten.

In den Figuren 5 und 6 sind ferner Auskragungen 27 nahe der Kontaktlinien 25, d.h. nahe den beiden zur Öffnung 16 hin orientierten Enden der Hypo-/Thenar-Abschnitte 13, 14 erkennbar. Diese Auskragungen 27 erhöhen die Stabilität im Bereich der (gedachten) Kontaktlinien 25, d.h. an dem Ort der Krafteinleitung in die zu behandelnden Hand.

In Figur 12.1 sind der distale Abschnitt 11 und der proximale Abschnitt 23 der Handmanschette 1 durch gestrichelte Kästen gekennzeichnet.

In Figur 12.2 sind der Dorsal-Abschnitt 4 sowie der Palmar-Abschnitt 5 gekennzeichnet, wobei letzterer sich in die beiden Hypo-/Thenar-Abschnitte 13, 14 unterteilt.

Die in Figur 13 dargestellte sehr einfach gebaute Ausführungsform einer erfindungsgemässen Handmanschette 1 besteht entweder aus einem flexiblen oder aus einem starren Material. In jedem Fall ist der Abstand 8 zwischen Dorsal-Abschnitt 4 und Palmar-Abschnitt 5 und gegebenenfalls das flexible Material so gewählt, dass die Hand beim Einschieben etwas gequetscht wird. Der so erzeugte Druck, welcher über die Hypo/Thenar-Abschnitte 13, 14 bzw. insbesondere über die gedachten Kontaktlinien 25 auf die Palmar-Seite der Hand weitergegeben wird, bewirkt auf die bereits beschriebene Weise die gewünschte Weitung des Karpaltunnels.

Ist die in Figur 13 dargestellte Handmanschette 1 aus einem flexiblen Material gefertigt, so wird der Abstand 8 zwischen Dorsal-Abschnitt 4 und Palmar-Abschnitt 5 beim Einschieben der Hand vergrössert, was wiederum eine Rückstellkraft erzeugt, mit welcher die Hypo/Thenar-Abschnitte 13, 14 den vorgenannten Druck an den Kontaktlinien 25 in Wirkrichtung 19 auf die Hand 2 ausüben.

Für Handmanschetten 1 mit Druckaufbau-Element 17 gemäss den Figuren 2 bis 12 und 15 bis 17 gilt: Nach dem Einstecken der Hand 2 in die Handmanschette 1 übt - je nach Handgrösse - auch ohne Aufblasen des Druckaufbau-Elements 17 der Dorsal-Abschnitt 4 bereits einen Druck auf die Dorsalseite der Hand aus. Dieser Druck ist in Figur 11 durch einen einzelnen Pfeil 19 oberhalb des Dorsalabschnitts 4 dargestellt. Ferner üben - ebenfalls je nach Handgrösse - auch die beiden Hypo-/Thenar-Abschnitte 13, 14, insbesondere an den Kontaktlinien 25, bereits einen gewissen Druck auf die Palmarseite der Hand aus. Dieser muss anfangs noch nicht zwangsläufig schräg, d.h. in Richtung der beiden Pfeile 19 unterhalb der Handmanschette 1 in Figur 11 verlaufen, sondern kann auch in Gegenrichtung zum Pfeil 19 oberhalb der Handmanschette 1 in Figur 11 und parallel zu diesem Pfeil 19 (also sozusagen "nach oben") verlaufen.

Wird das Druckaufbau-Element 17 vergrössert (meist also: wird das Luftkissen aufgeblasen), so vergrössert sich der Druck, welchen der Dorsal-Abschnitt 4 in Richtung des oberhalb der Handmanschette 1 in Figur 11 gezeigten Pfeils 19 auf die Hand ausübt. Bei zunehmendem Aufblasen des Druckaufbau-Elements 17 liegen die beiden Hypo-/Thenar-Abschnitte 13, 14 vorzugsweise derart eng an der Palmarseite der Hand 2 an, dass sich der Abstand 15 bei noch weiterem Aufblasen des Druckaufbau-Elements 17 vergrössert. Im Ergebnis stellen sich dann die unterhalb der Handmanschette 1 in Figur 11 angedeuteten Wirkrichtungen 19 der Hypo-/Thenar-Abschnitte 13, 14 ein, welche an den Kontaktlinien 25 auf die Palmarseite der Hand wirken. Da sie beidseits des Karpaltunnels angreifen, wird dieser wie gewünscht geweitet.

Es sei zu allen Ausführungsformen der Handmanschette 1 bemerkt, dass der Führungssteg 12 nicht zwingend über das distale Ende 9 hinaus ragen muss. Dieses Hinausrage bewirkt, dass die Länge des Grundkörpers 3 kurz gehalten werden kann, die Finger aber durch den verlängerten Führungssteg 12 dennoch wirksam geführt werden. Der Führungssteg 12 könnte aber auch kürzer ausgebildet sein, als in den Figuren dargestellt, auch ein kurzer Führungssteg 12 kann die Finger ausreichend wirksam führen. Insbesondere ist es auch denkbar, dass er sich vollständig im Inneren der Handmanschette 1 befindet. Ferner kann in sämtlichen Ausführungsformen auch auf den Führungssteg 12 verzichtet werden.

Die vierte Ausführungsform einer Handmanschette 1 ist in den Figuren 14.1 und 14.2 in verschiedenen Perspektiven gezeigt. Sie unterscheidet sich von der Handmanschette 1 nach Figur 13 dadurch, dass der Führungssteg 12 flexibel gestaltet ist. Der Führungssteg 12 gemäss den Figuren 14.1 und 14.2 kann sich in hypo-/thenar-Richtung, also sozusagen nach links oder rechts, krümmen. Die Hypo-/Thenar-Richtung sowie die Dorsal- und Palmar-Richtungen sind in Bezug auf Figur 14 durch Pfeile angedeutet.

Der Vorteil eines flexiblen Führungsstegs 12 liegt darin, dass die Handmanschette 1 zwar symmetrisch gestaltet ist und beide Hände eines Benutzers aufnehmen kann, die linke und die rechte Hand jedoch meist nicht vollständig symmetrisch ist. Die gemeinsame Breite von Mittelfinger und Zeigefinger ist meist etwas grösser ist als die gemeinsame Breite von Ringfinger und kleinem Finger. Dies kann dazu führen, dass der Mittelfinger und der Zeigefinger zwischen Führungssteg 12 und Seitenteil 24 etwas eingequetscht werden, während Ringfinger und kleiner Finger zwischen Führungssteg 12 und dem gegenüberliegenden Seitenteil 24 sehr locker liegen. Der flexible Führungssteg 12 ermöglicht es dem Mittelfinger, den Führungssteg 12 ein Stück weit in hypo-/thenar-Richtung zum Ringfinger zu drücken, wodurch Mittel- und Zeigefinger mehr Platz haben.

Die Flexibilität kann einerseits durch die Breite bzw. Dicke des Führungsstegs 12 gewährleistet werden. Je dünner der Führungssteg 12, desto leichter lässt er sich krümmen bzw. biegen. Zusätzlich oder alternativ kann die in Figur 14 gezeigte Auswölbung 28 vorgesehen sein, welche eine (entlang der dorsal-palmar-Achse gemessene) Länge des Führungsstegs 12 bei ansonsten unveränderter Höhe des Grundkörpers 3 erhöht, weil der Hebel, an welchem der Mittelfinger angreift, grösser wird als bei der Handmanschette 1 gemäss

Figur 13.

Es sind alternative Ausgestaltungen denkbar, um den Führungssteg 12 in hypo-/thenar-Richtung flexibel bzw. beweglich auszugestalten. Dies kann über Werkstoffauswahl, Abmessungen, Einbau von Gelenken, etc. geschehen.

Die Ausgestaltung umfassend einen flexiblen Führungssteg 12 mit Auswölbung 28 gemäss den Figuren 14.1 und 14.2 ist optionale und kann sowohl in Verbindung mit Handmanschetten 1 ohne Luftkissen 17 (gemäss Figuren 1 und 13), als auch in Verbindung mit Handmanschetten 1 mit Luftkissen 17 (gemäss Figuren 2 bis 12 und 15 bis 17) eingesetzt werden.

Ein Vergleich der Figuren 1 und 5 zeigt, dass eine linienförmige Druckeinleitung in die Hand auf verschiedene Weise bewerkstelligt werden kann. In der Ausführungsform gemäss Figur 1 bewirken die speziell geformten Abschnitte 20 die linienförmige Druckeinleitung, was durch die (gedachten) Kontaktlinien 25 in Figur 1 angedeutet ist. In der Ausführungsform gemäss den Figuren 2 bis 17 erfolgt die linienförmige Druckeinleitung hingegen durch die Formgebung der Hypo-/Thenar-Abschnitte 13, 14, insbesondere durch deren konvexe Abschnitte 26. Diese Formgebung bewirkt, dass der Kontakt zwischen Palmar-Abschnitt 5 und Hand vorrangig oder ausschliesslich über die Kontaktlinien 25 erfolgt.

Natürlich sind weitere Möglichkeiten denkbar, um eine linienförmige Druckeinleitung zu gewährleisten. Insbesondere sind alternativ geformte speziell geformte Abschnitte 20, eine alternative Formgebung oder eine Kombination aus beidem denkbar.

Die erhöhte Stabilität, welche in den Ausführungsbeispielen gemäss den Figuren 2 bis 17 durch die Auskragungen 27 bewirkt wird, kann natürlich auch auf andere Weise erzeugt werden. Es kann beispielsweise an eine erhöhte Steifigkeit des Werkstoffs, ggf. nur im Bereich nahe der Öffnung 16, gedacht sein. Weiterhin kann auch an Einlagen aus Metall oder einem anderen Stabilität verleihenden Material, an Rippen oder dergleichen gedacht sein. Ferner kann es Ausführungsbeispiele geben, bei denen die intrinsische Stabilität des Grundkörpers ausreicht und keine Massnahmen zur Erhöhung der Stabilität nötig sind.

Ausgehend von der in Figur 16 dargestellten Situation kann der Benutzer durch Betätigen der integrierten Pumpe 32, welche analog dem Pumpball 29 funktioniert, das Druckaufbau-Element 17 aufpumpen, wobei er den Druck mit Hilfe des Manometers 30 kontrollieren kann. Hierfür steht die integrierte Pumpe 32 fluidtechnisch mit dem Druckaufbau-Element 17 in Verbindung, wie dies auch in der Ausführungsform gemäss Figur 15 für den Pumpball 29 und das Druckaufbau-Element 17 gilt. Ein Ventil, mit dem der Druck nach Beendigung der Behandlung aus dem Druckaufbau-Element 17 abgelassen werden kann, ist vorgesehen, in den Figuren 16 und 17 jedoch nicht erkennbar.

### Weiß, Arat & Partner mbB

### Patentanwälte und Rechtsanwalt

### European Patent Attorneys

Aktenzeichen: P 5742/II-EP Datum: 10.09.2021

### Bezugszeichenliste

| | |
|---|---|
| 1 | Handmanschette |
| 2 | Hand |
| 3 | Grundkörper |
| 4 | Dorsal-Abschnitt |
| 5 | Palmar-Abschnitt |
| 6 | Schlitz |
| 7 | Daumen |
| 8 | Abstand zwischen dem Dorsal-Abschnitt und Palmar-Abschnitt |
| 9 | Distales Ende |
| 10 | Proximales Ende |
| 11 | Distaler Abschnitt |
| 12 | Führungssteg |
| 13 | erster Hypo-/Thenar-Abschnitt |
| 14 | zweiter Hypo-/Thenar-Abschnitt |
| 15 | Abstand zwischen erstem und zweitem Hypo-/Thenar-Abschnitt |
| 16 | Öffnung |
| 17 | Druckaufbau-Element |
| 18 | Breite |
| 19 | Wirkrichtung des Drucks |
| 20 | Speziell geformte Abschnitte |
| 21 | Schlauch |
| 22 | Distales Schlitz-Ende |
| 23 | Proximaler Abschnitt |
| 24 | Seitenteil |
| 25 | Kontaktlinie |
| 26 | Konvexer Abschnitt |
| 27 | Auskragung |
| 28 | Auswölbung |
| 29 | Pumpball |
| 30 | Manometer |
| 31 | Handbetätigtes Ventil |
| 32 | Integrierte Pumpe |
| 33 | |

## Patentansprüche

1. Handmanschette (1) zur Behandlung des Karpaltunnelsyndroms einer Hand (2),
die Handmanschette (1) umfassend einen Grundkörper (3),
wobei der Grundkörper (3) einen Dorsal-Abschnitt (4) und einen Palmar-Abschnitt (5) umfasst,
wobei der Dorsal-Abschnitt (4) eingerichtet ist, um an zumindest einer Stelle einen Druck auf die Dorsalseite der Hand (2) auszuüben,
wobei der Palmar-Abschnitt (5) eingerichtet ist, beidseits des Karpaltunnels jeweils an zumindest einer Stelle einen Druck auf die Palmarseite der Hand (2) auszuüben,
wobei der Dorsal-Abschnitt (4) und der Palmar-Abschnitt (5) gemeinsam eingerichtet sind, um durch die Ausübung des Drucks den Karpaltunnel der Hand (2) zu weiten,
wobei die Handmanschette (1) ein distales Ende (9) und ein proximales Ende (10) aufweist, wobei das proximale Ende (10) in Gebrauchslage das Handgelenk-seitige Ende der Handmanschette (1) ist,
**dadurch gekennzeichnet, dass**
der Grundkörper (3) zwischen dem Dorsal-Abschnitt (4) und dem Palmar-Abschnitt (5) zwei einander im Wesentlichen gegenüberliegende einseitig offene Schlitze (6) aufweist, deren offenen Enden am proximalen Ende (10) der Handmanschette (1) liegen, wobei die Schlitze (6) eingerichtet sind, den Daumen (7) der linken oder der rechten Hand (2) aufzunehmen.

2. Handmanschette (1) nach Anspruch 1, **dadurch gekennzeichnet, dass** ein Abstand (8), welcher zwischen dem Dorsal-Abschnitt (4) und dem Palmar-Abschnitt (5) gemessen wird, sich von einem distalen Ende (9) zu einem proximalen Ende (10) des Grundkörpers (3) hin vergrössert.

3. Handmanschette (1) nach einem der vorhergehenden Ansprüche, **gekennzeichnet durch** zumindest einen Führungssteg (12) in einem distalen Abschnitt (11) des Grundkörpers (3), wobei der zumindest eine Führungssteg (12) eingerichtet ist, um in einer Gebrauchslage zwischen zwei Fingern der Hand (2) zu liegen, und somit eine zur Behandlung wirksame Orientierung der Hand (2) zu gewährleisten.

4. Handmanschette (1) nach einem der vorhergehenden Ansprüche, **dadurch gekennzeichnet, dass** der Grundkörper (3) derart federnd eingerichtet ist, dass sich beim Einschieben der Hand (2) der Abstand (8) zwischen dem Dorsal-Abschnitt (4) und dem Palmar-Abschnitt (5) vergrössert, wodurch in Gebrauchslage der Druck zur Weitung des Karpaltunnels bereitgestellt wird.

5. Handmanschette (1) nach einem der vorhergehenden Ansprüche, **dadurch gekennzeichnet, dass** der Palmar-Abschnitt (5) einen ersten Hypo-/Thenar-Abschnitt (13) und einen zweiten Hypo-/Thenar-Abschnitt (14) umfasst, deren Abstand (15) sich verändern kann.

6. Handmanschette (1) nach Anspruch 5, **gekennzeichnet durch** eine Öffnung (16) zwischen dem ersten Hypo-/Thenar-Abschnitt (13) und dem zweiten Hypo-/Thenar-Abschnitt (14).

7. Handmanschette (1) nach zumindest einem der vorhergehenden Ansprüche, **gekennzeichnet durch** ein Druckaufbau-Element (17) am Dorsal-Abschnitt (4), wobei das Druckaufbau-Element (17) eingerichtet ist, an zumindest einer Stelle Druck auf die Dorsalseite der Hand (2) auszuüben.

8. Handmanschette (1) nach zumindest einem der vorhergehenden Ansprüche, **gekennzeichnet durch** einen einstückigen Grundkörper (3).

9. Handmanschette (1) nach zumindest einem der vorhergehenden Ansprüche, **dadurch gekennzeichnet, dass** der Grundkörper (3) im Profil eine C-Form aufweist.

10. Handmanschette (1) nach zumindest einem der vorhergehenden Ansprüche, **dadurch gekennzeichnet, dass** der Grundkörper (3) aus Kunststoff oder einem anderen elastischen Material gefertigt ist.

11. Handmanschette (1) nach zumindest einem der vorhergehenden Ansprüche, **dadurch gekennzeichnet, dass** eine Breite (18), welche in thenar-hypothenar-Richtung gemessen wird, sich von einem distalen Ende (9) zu einem proximalen Ende (10) des Grundkörpers (3) hin vergrössert.

## Claims

1. Hand cuff (1) for treating the carpal tunnel syndrome of a hand (2),
the hand cuff (1) comprising a base body (3),
wherein the base body (3) comprises a dorsal section (4) and a palmar section (5),
wherein the dorsal section (4) is adapted to exert a pressure on the dorsal side of the hand (2) at at least one location,
wherein the palmar section (5) is adapted to exert a pressure at at least one location on the palmar side of the hand (2) on both sides of the carpal tunnel,
wherein the dorsal section (4) and the palmar section (5) are jointly designed to widen the carpal tunnel of the hand (2) by the exertion of the pressure,
wherein the hand cuff (1) has a distal end (9) and a proximal end (10), wherein the proximal end (10) is the wrist-side end of the hand cuff (1) in the position of use,
**characterized in that**
the base body (3) has two substantially opposite one-sided open slots (6) between the dorsal section (4) and the palmar section (5), the open ends of which are located at the proximal end (10) of the hand cuff (1), wherein the slots (6) are designed to accommodate the thumb (7) of the left or right hand (2).

2. Hand cuff (1) according to claim 1, **characterized in that** a distance (8) measured between the dorsal section (4) and the palmar section (5) increases from a distal end (9) towards a proximal end (10) of the base body (3).

3. Hand cuff (1) according to at least one of the previous claims, **characterized by** at least one guide web (12) in a distal section (11) of the base body (3), the at least one guide web (12) being configured to lie, in a position of use, between two fingers of the hand (2), thereby ensuring an orientation of the hand (2) effective for treatment.

4. Hand cuff (1) according to at least one of the previous claims, **characterized in that** the base body (3) is resiliently arranged in such a way that, when inserting the hand (2), the distance (8) between the dorsal section (4) and the palmar section (5) increases, as a result of which the pressure for widening the carpal tunnel is provided in the position of use.

5. Hand cuff (1) according to one of the previous claims, **characterized in that** the palmar section (5) comprises a first hypo/thenar section (13) and a second hypo/thenar section (14) whose distance (15) can vary.

6. Hand cuff (1) according to claim 5, **characterized by** an opening (16) between the first hypo-/thenar section (13) and the second hypo-/thenar section (14).

7. Hand cuff (1) according to at least one of the previous claims, **characterized by** a pressure-building element (17) on the dorsal section (4), the pressure-building element (17) being configured to exert pressure at at least one location on the dorsal side of the hand (2).

8. Hand cuff (1) according to at least one of the previous claims, **characterized by** a one-piece base body (3).

9. Hand cuff (1) according to at least one of the previous claims, **characterized in that** the base body (3) has a C-shape in profile.

10. Hand cuff (1) according to at least one of the previous claims, **characterized in that** the base body (3) is made of plastic or another elastic material.

11. Hand cuff (1) according to at least one of the previous claims, **characterized in that** a width (18), which is measured in the thenar-hypothenar direction, increases from a distal end (9) to a proximal end (10) of the base body (3).

## Revendications

1. Manchette (1) pour la main, destinée au traitement du syndrome du canal carpien d'une main (2),
la manchette (1) comprenant un corps de base (3),
le corps de base (3) comprenant une partie dorsale (4) et une partie palmaire (5),
la partie dorsale (4) étant conçue pour exercer une pression sur la face dorsale de la main (2) à au moins un endroit,
la partie palmaire (5) étant conçue pour exercer une pression sur la face palmaire de la main (2) au moins à un endroit de chaque côté du canal carpien,
la partie dorsale (4) et la partie palmaire (5) étant conçues conjointement pour élargir le canal carpien de la main (2) en exerçant la pression,
la manchette (1) présentant une extrémité distale (9) et une extrémité proximale (10), l'extrémité proximale (10) étant, en position d'utilisation, l'extrémité côté poignet de la manchette (1),
**caractérisée en ce que**
le corps de base (3) comporte, entre la partie dorsale (4) et la partie palmaire (5), deux fentes ouvertes (6) sensiblement opposées l'une à l'autre, dont les extrémités ouvertes se trouvent à l'extrémité proximale (10) de la manchette (1), les fentes (6) étant conçues pour recevoir le pouce (7) de la main gauche ou droite (2).

2. Manchette (1) pour la main selon la revendication 1,
**caractérisée en ce qu'**une distance (8) mesurée entre la partie dorsale (4) et la partie palmaire (5) augmente d'une extrémité distale (9) vers une extrémité proximale (10) du corps de base (3).

3. Manchette (1) pour la main selon l'une des revendications précédentes, **caractérisée par** au moins une barrette de guidage (12) dans une partie distale (11) du corps de base (3), ladite au moins une barrette de guidage (12) étant conçue pour se trouver, en position d'utilisation, entre deux doigts de la main (2) et pour assurer ainsi une orientation de la main (2) efficace pour le traitement.

4. Manchette (1) pour la main selon l'une des revendications précédentes, **caractérisée en ce que** le corps de base (3) est conçu de manière élastique de telle sorte que, lors de l'insertion de la main (2), la distance (8) entre la partie dorsale (4) et la partie palmaire (5) augmente, ce qui, en position d'utilisation, fournit la pression nécessaire à l'élargissement du canal carpien.

5. Manchette (1) pour la main selon l'une des revendications précédentes, **caractérisée en ce que** la partie palmaire (5) comprend une première partie hypo-/thénar (13) et une deuxième partie hypo-/thénar (14) dont la distance (15) peut varier.

6. Manchette (1) pour la main selon la revendication 5,
**caractérisée par** une ouverture (16) entre la première partie hypo-/thénar (13) et la deuxième partie hypo-/thénar (14).

7. Manchette (1) pour la main selon l'une au moins des revendications précédentes,
**caractérisée par** un élément d'établissement de pression (17) situé sur la partie dorsale (4), l'élément d'établissement de pression (17) étant conçu pour exercer une pression sur la face dorsale de la main (2) à au moins un endroit.

8. Manchette (1) pour la main selon l'une au moins des revendications précédentes,
**caractérisée par** un corps de base monobloc (3).

9. Manchette (1) pour la main selon l'une au moins des revendications précédentes,
**caractérisée en ce que** le corps de base (3) présente un profil en forme de C.

10. Manchette (1) pour la main selon l'une au moins des revendications précédentes,
**caractérisée en ce que** le corps de base (3) est fabriqué en matière plastique ou en un autre matériau élastique.

11. Manchette (1) pour la main selon au moins l'une des revendications précédentes,
**caractérisée en ce qu'**une largeur (18), mesurée dans la direction thénar-hypothénar, augmente d'une extrémité distale (9) vers une extrémité proximale (10) du corps de base (3).
